(19) Europäisches Patentamt
European Patent Office
Office européen des brevets

(11) Publication number: **0 133 354**
**B1**

(12) # EUROPEAN PATENT SPECIFICATION

(45) Date of publication of patent specification: **25.01.89**

(51) Int. Cl.$^4$: **A 61 K 7/30**

(21) Application number: **84305043.6**

(22) Date of filing: **25.07.84**

(54) **Denture cleansing compositions.**

(30) Priority: **09.08.83 GB 8321404**

(43) Date of publication of application:
**20.02.85 Bulletin 85/08**

(45) Publication of the grant of the patent:
**25.01.89 Bulletin 89/04**

(84) Designated Contracting States:
**AT BE CH DE FR GB IT LI NL SE**

(56) References cited:
**EP-A-0 027 693**
**FR-A-2 515 683**
**FR-A-2 522 675**
**GB-A-2 096 162**
**US-A-4 180 467**

(73) Proprietor: **Interox Chemicals Limited**
**3 Bedford Square**
**London WC1B 3RA (GB)**

(72) Inventor: **Banks, Norman Edward**
**6, Radlett Close Penketh**
**Warrington Cheshire (GB)**
Inventor: **Hignett, Geoffrey John**
**8, Brookside Avenue Statham**
**Lymm Cheshire (GB)**
Inventor: **Smith, Eileen**
**6, The Stables Station Lane**
**Guilden Sutton Chester Cheshire (GB)**

(74) Representative: **Pearce, Timothy et al**
**Laporte Industries Limited Group Patent**
**Department P.O. Box 2 Moorfield Road**
**Widnes Cheshire WA8 0JU (GB)**

**EP 0 133 354 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to denture cleansing compositions especially in tablet form, and in particular to such tablets which contain an organic peroxygen compound.

Denture cleansing compositions in tablet and powder form are known, and they normally comprise a source of effervescence, an oxidising agent and an acidic or alkaline buffer, depending upon the type of composition selected, of which two or more functions can be performed by the same component. The compositions are often compacted to form tablets which offers a simple means of providing a measured dose of the active ingredients to the user. The tablets are dissolved in an aqueous bath containing the dentures, whereupon it is the intention that stains are oxidised and small particles such as food particles are swept by the effervescence into suspension. Compositions of this type are described for example in US—A—4 180 467 (Barth—Colgate Palmolive).

It will be recognised that although it is inherently desirable to employ a powerful oxidising agent and an effective source of effervescence, there can be significant problems of bringing them together in usable form. It is further recognised that as a class percarboxylic acids are capable oxidising agents but as a general rule suffer from one or more of the difficulties, in the context of a denture cleansing composition, of being in the wrong physical form, many of them being liquid at or near ambient storage temperatures, or are unstable to physical or thermal shock or inherently progressively and rapidly lose their oxidising activity, either by autocatalysis or externally induced decomposition, or are only poorly or slowly soluble in aqueous media employed by the end user. Clearly each of the problems outlined above is of importance in the context of providing a denture cleansing tablet. The tablet during formation is subjected to considerable pressure, and can also be heated, is often stored for a matter of months before use and an insoluble agent neither performs very well nor meets with approval from the end user. Thus, it will be fully understood that even though there have been many formulations described hitherto using inorganic peroxygen compounds as the oxidising agent, it is not considered practical with expectation of success merely to substitute an organic peroxygen compound for the inorganic peroxygen compounds in such formulations.

Surprisingly, it has been found that there exists a class of organic peroxygen compounds containing a peroxyacid group which are sufficiently stable for them to be incorporated safely in tablets containing the other ingredients of denture cleansing compositions, and show acceptable resistance to decomposition during storage coupled with demonstrable solubility in aqueous media over a wide range of acidity/alkalinity.

According to the present invention there is provided a denture cleansing composition especially in tablet form comprising

(1) at least 10% by weight of a particulate water-soluble effervescence generator or generator system,

(2) sufficient net acid or alkali in particulate form to obtain a desired solution pH in water, especially one selected in the range of pH 2 to pH 12 upon dissolution of the composition,

(3) at least 1% by weight of a particulate hydrated magnesium salt of a substituted peroxygen compound having the empirical (anhydrous) formula:

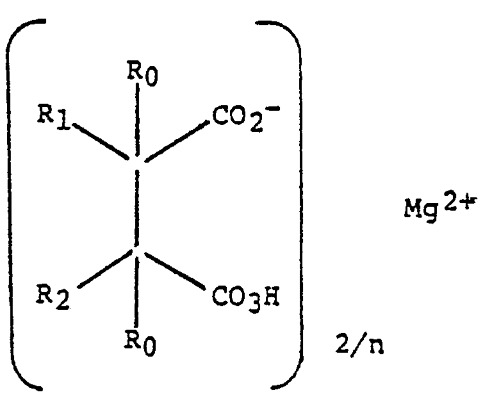

in which $R_0$ each represent hydrogen or both combine together to form an olefinic bond and $R_1$ and $R_2$ combine together with each other and the carbon atoms from which they depend to form a carbocyclic nucleus, which nucleus may optionally be further substituted by one or more of the functional groups selected from alkyl, carboxylate, percarboxylic acid, sulphonate, nitro, chloro and bromo groups;
or in which $R_1$ and $R_2$ each represent either hydrogen or an alkyl group and both $R_0$ combine together to form an olefinic bond;
and n represents the number of carboxylate groups present in the compound.

Advantageously it has been found that it is practicable to make and store such tablets which offer an organic water soluble peroxyacid bleach even in alkaline solution, and this is rendered possible by the selection of the specified magnesium salts. It is especially surprising that the overall system functions in

that it would reasonably be expected that on dissolution of the magnesium salt, the peroxyacid would be available for reaction with the effervescence generator or alkalising components of the composition to generate water-insoluble products which would not only appear unacceptable to the user but would impair performance. To a certain extent, a related effect does become noticeable under the higher alkaline conditions and the amelioration of the effect will be discussed subsequently herein.

The effervescence generator (system) conveniently is selected to generate either carbon dioxide or oxygen, or both, possibly selected in conjunction with the desired acidity/alkalinity of the eventual denture soak solution. At a pH of up to about pH 10, the system conveniently can comprise in combination a solid water soluble carbonate or bicarbonate with a water-soluble solid organic acid. The water-soluble carbonate/bicarbonate salts are preferably alkali metal salts, and in particular the sodium or potassium salts. It is often convenient to select, where practicable the anhydrous or lower hydrated salt in preferance to the higher hydrated salt, although all are usable. The most convenient salts, are sodium carbonate, anhydrous or monohydrate, sesquicarbonate and bicarbonate, and other salts include potassium carbonate, anhydrous and dihydrate. As an alternative part at least of the carbonate can be present as the hydrogen peroxide adduct the so called sodium percarbonate. The water soluble organic acid is often selected from tartaric acid, citric acid, lactic acid, succinic acid, glutaric acid, maleic acid, fumaric acid and malonic acid. Alternatively all or part of the acid component can be provided by a water-soluble solid inorganic acid or acid salt, of which particularly suitable examples include sulphamic acid and alkali metal bisulphates such as the sodium or potassium salts or a mixture thereof. These inorganic acids/salts are especially useful at generating a pH in the region of pH 4 or lower.

An alternative or additional effervescence generator, employable generally in tablets throughout the pH range but normally as the principal generator at above pH 10, is a releaser of molecular oxygen such as, in particular, anhydrous sodium perborate. At below pH 10 its proportion of the generator system does range from 0—90%.

The proportion of the effervescence generator/system is normally up to 90% by weight of the total of the composition and often from 10 to 40% specifically for that purpose. In the case of the acid/carbonate system it will be recognised that the pH of the resultant solution can easily be varied by changing the ratio of acid to carbonate in the composition. Thus, when the acid/carbonate system is employed in non-equimolar proportions, the excess of the one or the other is contributing in effect as or as a part of component (2). In practice, when using such a two part system, the acid and the carbonate each often comprise from 5 to 20% by weight in equimolar amounts for the purpose of generating carbon dioxide and further amounts of either part can be included for pH adjustment.

In addition to employing non-equimolar proportions of the various acid and carbonate compounds mentioned previously or any one of them alone, the pH can be adjusted by a separate agent of component (2) comprising one or more water soluble alkali metal silicates, phosphates or borates. These include in particular sodium metasilicates, and orthosilicates and waterglass, sodium hexametaphosphate, sodium dihydrogen orthophosphate, and sodium metaborate. Useful potassium salts include the meta and tetrasilicates, orthometa and pyrophosphates and metaborates. Naturally mixtures of any two or more of these salts can be used, or a mixture of one or more of such salts with excess carbonate/bicarbonate can also be used. By using such compounds in appropriate amounts, either alone or in combination with each other and/or the acid/carbonate system, it is possible to generate any desired solution pH within the specified range of pH2 to pH12.

It is particularly useful to employ the non-carbonate salts in a composition intended for generation of an alkaline solution and especially for generation above pH 9. In practice, therefore, it is likely to follow that such salts are employed in conjunction with an oxygen gas effervescence generator, e.g. anhydrous sodium perborate, but the carbon dioxide generating system can of course also be used. Ignoring any contribution from excess carbonate, the alkali salts are present in many invention compositions to a total amount of from 0 to 75% by weight of the composition and often from 0 to 40%. For compositions intended to generate a pH of over 10 the amount of especially metasilicates and similar alkaline agents is often at least 5% and commonly from 10% to 40% w/w.

It will be recognised that where reference is made herein to the magnesium salt of a particular peroxygen compound, the salt is formed from the carboxylic acid group and not the peroxycarboxylic acid substituent so that the latter remains intact. Moreover, the salt is in practice in hydrated form. The carbocyclic nucleus can be cycloaliphatic or aromatic, but of course when it is aromatic both $R_0$ groups have combined to generate one degree of olefinic unsaturation. Moreover, it will be recognised that where $R_1$ and $R_2$ combine to produce a carbocyclic nucleus which is substituted additionally by a functional group described herein, the resultant product can in general be a mixture of isomers particularly when the peroxygen compound has been obtained by hydrogen peroxide oxidation of the corresponding anhydride. Thus, for example, the compound obtained by reacting hydrogen peroxide and magnesium oxide with trimellitic anhydride is a mixture containing, it is believed, benzene-1,3-dicarboxylate-4-peroxycarboxylic acid and benzene-1,4-dicarboxylate-3-peroxycarboxylic acid. Further examples include the product obtained from pyromellitic anhydride by oxidation with hydrogen peroxide and neutralisation with a magnesium base. Once again the product is believed to be a mixture of isomers comprising benzene-1,4-dicarboxylate-2,5-diperoxycarboxylic acid, and benzene-1,5-dicarboxylate-2,4-diperoxycarboxylic acid. Thus, the products derived from trimellitic and pyromellitic anhydrides are both salts in which the benzene

nucleus is substituted by one or more further carboxylate groups, so that n in the formula is correspondingly 2 or more for them.

Desirably, where the benzene nucleus is further substituted by a nitro, chloro or bromo substituent, the substituent is para to either the carboxylate substituent or the peroxycarboxylic acid substituent, and that in practice, where the product is obtained from the corresponding anhydride, a mixture of both isomers is likely to occur. It will be understood that in such reactions, the relative positions of the additional substituent and the carbonyl groups originally forming part of the anhydride group and later forming respectively the carboxylate and peroxycarboxylic acid substituents does not change and the two isomers arise merely as a result of the asymmetry of the molecule. Where the additional substituent in the carbocyclic nucleus is an alkyl group, it can be a short chain alkyl, for example methyl, ethyl or propyl up to a long chain hydrophobic substituent such as dodecyl, hexadecyl, or octadecyl substituents. Conveniently, the alkyl substituent can be either ortho or para to either the carboxylate or the peroxycarboxylic acid substituents.

One especially suitable and convenient member of the class of magnesium salts of the peroxy compounds is magnesium monoperoxyphthalate, by which we mean herein the compound having the empirical formula

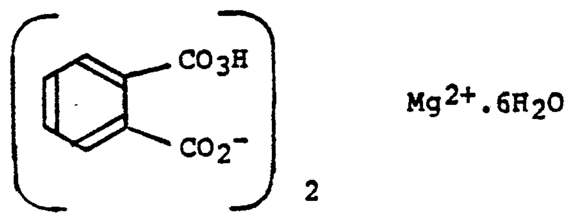

It will be recognised that the salt is that of the carboxylate group only, that the peroxy group remains in acid form and that n is 1. The aforementioned magnesium salt, which for the sake of brevity may be referred to herein alternatively as MMPP, demonstrates good storage stability itself, and in this respect MMPP typifies the magnesium salts of the peroxy compounds herein.

Some of the other salts included herein can be regarded as the aromatic compounds which have been hydrogenated. Examples of such compounds include the magnesium salt of cyclohex-4-ene-1-carboxylate-2-peroxycarboxylic acid. Preferably, the cycloaliphatic compounds are fully saturated as in the magnesium salt of cyclohexane-1-carboxylate-2-peroxycarboxylic acid, otherwise referred to as the magnesium salt of hexahydro-monoperoxyphthalate. Further examples of compounds include the analogues of hexahydro-monoperoxyphthalate (magnesium salts) further substituted in, for example, the 3 or 4 position by a substituent such as alkyl, nitro, carboxylate or sulphonate group.

Peroxy compounds in which $R_1$ and $R_2$ are either an alkyl group or hydrogen, it will be recognised, include magnesium monoperoxy-maleate and the corresponding compounds in which the olefinic group is substituted by one or two alkyl groups and in such compounds n is 1. The alkyl group when present can be methyl through to long chain such as octadecyl. The peroxycompounds normally contain up to 20 carbon atoms.

The magnesium salts of the peroxyacid/carboxylate compound can be prepared by the method described in EP—A—27693, the resultant crystalline material being hydrated, x-ray diffraction data indicating a hexahydro magnesium structure. Many of the salts, including the most preferred salt are preferably made by a process as described in EP—A—66992 both being to Interox Chemicals Limited.

In practice, the method of manufacture of MMPP and the other members of the class often results in the particules of the peroxygen compound containing a small proportion of the corresponding non-peroxygenerated compound. Thus for MMPP, a small proportion of magnesium phthalate can be present.

The magnesium salt is normally present in an amount of up to 70% by weight of the composition and in many embodiments from 2 to 50% particularly 5 to 35% by weight thereof.

As referred to briefly above, at high solution pHs such as above pH 9 there is an increased tendency for the magnesium salt constituting the oxidising agent to interact with carbonate released from the tablet or naturally present in the water used as solvent for the cleansing solution. This tends to manifest itself by either deposition of insoluble salts or production of a murky suspension or a retardation of the rate of disintegration of the tablet. This is a problem that hitherto has not needed consideration in view of the fact that inorganic peroxygen compounds have normally comprised the sodium salt, so that interaction with the alkaline salt component of the denture cleansing composition or hydrolysis does not lead to the production of water-insoluble salts. This problem can be ameliorated by incorporating within the tablet/composition one or more magnesium complexing agents, preferably in close juxtaposition with the oxidising agent and preferably in a weight ratio sufficient to complex all the magnesium ions. Such complexing agents tend to be salts, especially sodium, of soluble hydroxycarboxylic acids, such as sodium citrate, or sodium tartrate or the salts, especially sodium, of the various aminopolymethylene phosphonic acids of general formula $R_2N—(NR)_n—R$ where n is 0, 1 or 2 and R represents a methylene phosphonate group; as an alternative the potassium salts could likewise be used. It is convenient to use a weight of up to

one mole of complexing agent per mole of magnesium in the oxidising agent used. Excess complexing agent can be contemplated but at added expense for the formulation. Accordingly, the weight of complexing agent used in alkaline solution-generating compositions is often at least one eight part by weight of the magnesium salt bleaching agent and is often from one half to three times the weight of the bleaching agent. Expressed differently at least 1% of the complexing agent is conveniently present and often from 5 to 40% is present. It will be recognised that the complexing agent can be of corresponding value when the composition is present in some other solid form such as a granulate or powder mixture.

It is not essential for the aforementioned active ingredients to comprise substantially the entire composition. Tablets of certain standard sizes are less expensive to package than non-standard ones and indeed the user has an expectation of a particular size to provide a given performance. Were smaller ones to be used, there would be an enhanced tendency for wasteful multitablet dosing. The tablet can also contain up to 80% by weight of a water-soluble diluent which in practice is often an alkali metal salt of a mineral acid, such as anhydrous sodium sulphate or sodium chloride. It will be recognised that many water soluble salts that might otherwise be considered here such as various water soluble alkali metal borates or phosphates have been referred to in the context of an agent for pH adjustment or buffering. Alternative diluents include alkali metal, especially sodium or potassium water soluble salts of carboxylic acids such as phthalic acid, salicyclic acid and acetic acid.

The cleansing composition can also contain one or more minor ingredients of the types that hitherto have been suggested as being useful in denture cleansing tablets containing an oxidising agent. These minor ingredients typically are present in a total of not more than about 15% by weight of the compositions. Such ingredients can comprise one or more surfactants which can be anionic, nonionic, cationic, zwitterionic or amphoteric, often in an amount of 0.1 to 5% w/w to assist cleansing and wetting of the dentures, and can assist lubrication of the tablet including alkyl benzene sulphonates. It is often desirable to incorporate a small amount of a water-soluble binder, for example polyvinylpyrolidones, particularly those having a molecular weight of 5000 to 20000, or polyethyleneglycols, particularly those having a molecular weight of 3000 to 10000 and in an amount of 0.5 to 10% w/w, often 2 to 7% w/w. Other tabletting acids which can be included in minor amounts include talc, cellulose derivatives, gelatine, starch and methylcellulose derivatives. The disintegrating effect of the gas generating system can be assisted by inclusion of a disintegrant, typically swelling cross-linked polymers, often in an amount of up to 2% w/w, of which various commercial products are readily available including cross-linked poly(N-vinyl) pyrrolidones and carboxy-methylcellulose.

In practice, it is often convenient to incorporate one or more water soluble dyes, for example at 0.01 to 0.2% w/w particularly those which are bleached by a peroxygen compound in aqueous solution within a short period, often 10 to 30 minutes. Such dyes are known in the art, for example a range of azo dyes meet the criterion at the typical encountered cleansing solution pHs. In addition it is desirable often to include a flavour oil and/or a perfume to impart an attractive flavour to the denture and/or a pleasing scent to the tablet, where such flavour can be adsorbed into preferably solid soluble carriers. Flavour is often present at 0.05 to 0.5% w/w and perfume at below 0.1% w/w. A soluble sweetener can also be included, such as sugars and saccharine.

The compositions described herein can readily be made by premixing thoroughly the components thereof which are usually in powder or granule form in the desired proportions and passing the mixtures to conventional tabletting apparatus where binders and the like are employed that they can be sprayed into the mixture usually in solution and the resultant mixture where necessary subsequently dried.

The invention will now be further illustrated by the following non-limitative Examples.

## Examples 1 to 20

In each of Example compositions 1 to 20 formulations were prepared by dry mixing at ambient temperature the components in powder form together in the weight ratios specified in Table 1. The magnesium salt comprised magnesium monoperoxyphthalate hexahydrate having an avox content of 6% w/w. The Acid was succinic acid, and the Bicarb and Carb were respectively anhydrous sodium bicarbonate and sodium carbonate monohydrate. The added alkali, NMS was sodium metasilicate, and TNC represented the complexing agent, trisodium citrate and ANS anhydrous sodium sulphate. The compositions were further mixed with minor amounts of PEG 6000 as binder, SLS lubricant viz sodium lauryl sulphate and PVP disintegrant viz POLYPLASDONE XL cross-linked polyvinylpyrrolidone to a total of 7.2% w/w and formed into tablets each weighing about 4 to 4.2 g. Various of the tablets were tested and found to have a crushing strength in the region of 30—32 lbs (13 to 15 kg) and to dissolve readily in aqueous solution at 50°C, one tablet in 200 mls taking only just over a minute in mildly acidic conditions, about 2 minutes at pH 6 to 8.5 and progressively longer at higher pHs, in the absence of added sodium citrate, but between 1 and 2 minutes in the presence of the sodium citrate. Various of the formulations have been stored at 32°C in sealed containers to determine their avox stability. The proportion of the initial avox remaining after 6 weeks is listed as Avox % Left, except for Example 15 for which the measurement was made after 7 weeks. The letters nm indicate that the measurement was not made.

From Table 1, it can be seen that the compositions were able to be made and tabletted safely using an organic peroxyacid over a very wide range of pHs developed in the solution, and that the resultant product had a very good stability. The tablet dissolved quickly with evident gassing and rapid development of

bleaching agent in solution, rendering the solution extremely suitable for the cleansing of dentures. Where the compositions began to suffer from an impaired rate of dissolution, the problem was cured by incorporation of sodium citrate.

Table 1

| Ex. No. | Mag. Salt %w/w | Acid %w/w | $CO_2$ generator Bicarb %w/w | Carb %w/w | Alkali NMS %w/w | Complex Agent %w/w |
|---|---|---|---|---|---|---|
| 1 | 10 | 25 | 10 | - | - | - |
| 2 | 10 | 20 | 10 | - | - | - |
| 3 | 10 | 15 | 10 | - | - | - |
| 4 | 10 | 27 | - | 15 | - | - |
| 5 | 10 | 10 | 10 | - | - | - |
| 6 | 10 | 7 | 10 | - | - | - |
| 7 | 10 | 14 | - | 15 | - | - |
| 8 | 10 | 14 | - | 20 | - | - |
| 9 | 10 | 14 | - | 25 | - | - |
| 10 | 10 | 14 | - | 30 | - | - |
| 11 | 10 | 14 | - | 40 | - | - |
| 12 | 10 | 14 | - | 50 | - | - |
| 13 | 10 | 14 | - | 60 | - | - |
| 14 | 10 | 14 | - | 68.8 | - | - |
| 15 | 40 | 20 | 10 | - | - | - |
| 16 | 10 | 14 | - | 58.8 | - | 10 |
| 17 | 10 | 14 | - | 48.8 | - | 20 |
| 18 | 10 | 14 | - | 38.8 | - | 30 |
| 19 | 10 | 14 | - | 38.8 | - | 20 |
| 20 | 10 | 14 | - | 15 | 14 | 39.8 |

Table 1 Cont'd

| Ex. No. | Diluent ANS %w/w | pH of soln | Binder %w/w | SLS %w/w | PVP %w/w | Avox % Left |
|---|---|---|---|---|---|---|
| 1 | 47.8 | 4.1 | 6 | 0.2 | 1 | nm |
| 2 | 52.8 | 4.3 | 6 | 0.2 | 1 | nm |
| 3 | 57.8 | 4.5 | 6 | 0.2 | 1 | 90.2 |
| 4 | 40.8 | 4.7 | 6 | 0.2 | 1 | 91.3 |
| 5 | 62.8 | 5.0 | 6 | 0.2 | 1 | 91.8 |
| 6 | 65.8 | 5.4 | 6 | 0.2 | 1 | nm |
| 7 | 53.8 | 5.7 | 6 | 0.2 | 1 | nm |
| 8 | 48.8 | 6.3 | 6 | 0.2 | 1 | 90.4 |
| 9 | 43.8 | 7.2 | 6 | 0.2 | 1 | nm |
| 10 | 38.8 | 7.7 | 6 | 0.2 | 1 | 90.6 |
| 11 | 28.8 | 9.1 | 6 | 0.2 | 1 | 89.9 |
| 12 | 18.8 | 9.5 | 6 | 0.2 | 1 | nm |
| 13 | 8.8 | 9.75 | 6 | 0.2 | 1 | nm |
| 14 | 0 | 9.9 | 6 | 0.2 | 1 | 93.6 |
| 15 | 22.8 | 4.5 | 6 | 0.2 | 1 | 88.2 |
| 16 | - | 9.6 | 6 | 0.2 | 1 | nm |
| 17 | - | 9.4 | 6 | 0.2 | 1 | nm |
| 18 | - | 9.0 | 6 | 0.2 | 1 | nm |
| 19 | 10 | 9.0 | 6 | 0.2 | 1 | nm |
| 20 | - | 10 | 6 | 0.2 | 1 | nm |

Examples 21 to 24

Further compositions similar to those in the preceding Examples were similarly produced but in powder form. The compositions are summarised in Table 2. The headings are as in Table 1 except that the amounts are parts by weight that total 93. The pH shown is that of a 1.86% solution at 24°C in distilled water. The avox stability of the powders was tested at 32°C in sealed containers, and at 28°C and 40% relative humidity. After 10 weeks storage, many of the samples analysed to the same avox content as originally present and the worst had lost only 5.6%.

Table 2

| Ex No | Mag salt | Acid | $CO_2$ generator Bicarb | $CO_2$ generator Carb | Diluent ANS | pH of Soln |
|---|---|---|---|---|---|---|
| 21 | 20 | 20 | 10 | - | 1 | 4.4 |
| 22 | 30 | 20 | 10 | - | 43 | 4.2 |
| 23 | 20 | 14 | - | 40 | 19 | 9.2 |
| 24 | 30 | 14 | - | 45 | 4 | 9.2 |

Examples 25 to 30

Further compositions similar to those in Examples 21 to 24 were similarly produced, but employing inorganic acid/salts instead of an organic acid. The formulations compositions are summarised in Table 3

amounts shown being parts by weight. NSA represents sulphamic acid each of which compositions generates a pH in the region of pH 2. The avox stabilities of the formulations were likewise tested at 32°C in a sealed container or at 28°C/40% RH in an open container. Many of the samples again showed no detectable avox loss after 10 weeks storage and of the others, the worst loss was only 8%.

Table 3

| Ex No | Mag Salt | Acid/Salt | | Bicarb | Diluent |
|---|---|---|---|---|---|
| 25 | 20 | NSA | 20 | 10 | 43 |
| 26 | 30 | NSA | 20 | 10 | 33 |
| 27 | 20 | $NaHSO_4$ | 20 | 10 | 43 |
| 28 | 30 | $NaHSO_4$ | 20 | 10 | 33 |
| 29 | 20 | $KHSO_4$ | 20 | 10 | 43 |
| 30 | 30 | $KHSO_4$ | 20 | 10 | 33 |

Examples 31 to 34

Further Examples in powder form were prepared containing anhydrous sodium perborate (PBS), again by mixing at ambient temperature the ingredients in particulate form. The compositions of the formulations are summarised in Table 4. The pH of the solutions of Examples 31 and 34 was measured and found to be respectively 10.3 and 6.4. The diluent comprised mainly sodium sulphate but also included 0.2 parts of SLS. The acid was succinic acid.

Table 4

| Ex No | Mag Salt | PBS | Acid | Carb | Diluent |
|---|---|---|---|---|---|
| 31 | 20 | 14 | - | 40 | 20 |
| 32 | 20 | 20 | - | - | 53 |
| 33 | 20 | 20 | - | 7 | 46 |
| 34 | 20 | 10 | 10 | 3 | 50 |

**Claims**

1. A denture cleansing composition comprising:—

(1) at least 10% by weight of a particulate water-soluble effervescence generator or generator system,

(2) sufficient net acid or alkali in particulate form to obtain a desired solution pH in water, especially one selected in the range of pH 2 to pH 12 upon dissolution of the composition,

(3) at least 1% by weight of a particulate hydrated magnesium salt of a substituted peroxygen compound having the empirical (anhydrous) formula:

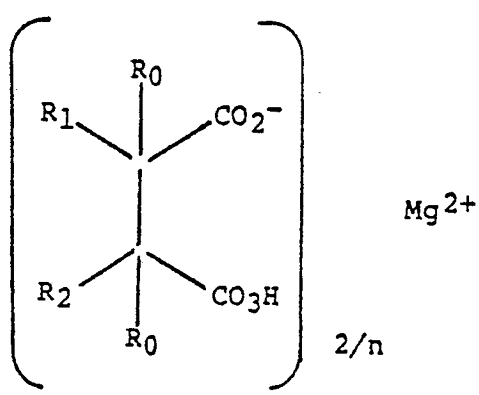

in which $R_0$ each represent hydrogen or both combine together to form an olefinic bond and $R_1$ and $R_2$ combine together with each other and the carbon atoms from which they depend to form a carbocyclic nucleus, which nucleus may optionally be further substituted by one or more of the functional groups selected from alkyl, carboxylate, percarboxylic acid, sulphonate, nitro, chloro and bromo groups;
or in which $R_1$ and $R_2$ each represent either hydrogen or an alkyl group and both $R_0$ combine together to form an olefinic bond;
and n represents the number of carboxylate groups present in the compound.

2. A composition according to claim 1 characterised in that the effervescence generator system comprises a solid water-soluble carbonate or bicarbonate together with a solid water-soluble organic acid or inorganic solid acid or in organic acid salt.

3. A composition according to claim 1 or 2 characterised in that the acid is selected from succinic acid, sulphamic acid and an alkali metal bisulphate.

4. A composition according to claim 1 characterised in that the effervescence generator comprises an anhydrous sodium perborate.

5. A composition according to any preceding claim characterised in that the effervescence generator comprises 10 to 40% w/w of the composition.

6. A composition according to any preceding claim containing up to 40% w/w of a water-soluble alkali selected from water-soluble alkali metal silicates, phosphates, borates and carbonates.

7. A composition according to any preceding claim characterised in that the hydrated magnesium salt of the peroxygen compound comprises 5 to 35% w/w of the composition.

8. A composition according to any preceding claim characterised in that the hydrated magnesium salt is magnesium monoperoxyphthalate.

9. A composition according to any preceding claim characterised in that it contains sufficient alkali such that the resultant aqueous solution has a pH of above pH 9 and contains from 1 to 40% w/w of a soluble alkaline earth metal complexing agent.

10. A composition according to claim 9 characterised in that the complexing agent is selected from sodium citrate, sodium tartrate and amino poly (methylene phosphonic acid) salts.

**Patentansprüche**

1. Reinigungszusammensetzung für Zahnprothesen enthaltend:

(1) mindestens 10 Gew.-% eines teilchenförmigen wasserlöslichen Brauseeffekt-Erzeugers oder Brausseeffekt erzeugenden Systems,

(2) ausreichend reine Säure oder reines Alkali in Teilchenform, um einen gewünschten Lösungs-pH in Wasser zu erhalten, insbesondere einen im Bereich von pH 2 bis pH 12 nach Auflösung der Zusammensetzung,

(3) mindestens 1 Gew.-% eines teilchenförmigen hydratisierten Magnesiumsalzes einer substituierten Peroxyverbindung mit der empirischen (wasserfreien) Formel:

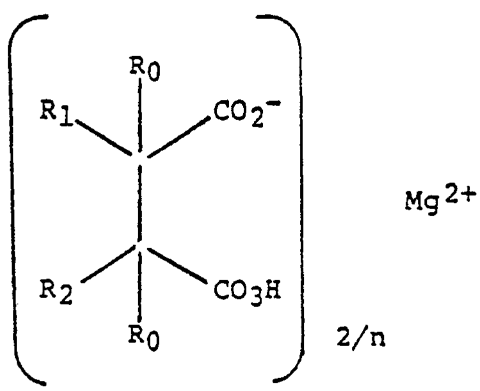

in der $R_0$ je Wasserstoff bedeuten oder beide zusammen eine olefinische Bindung bilden und $R_1$ und $R_2$ miteinander und den Kohlenstoffatomen, mit denen sie verbunden sind, einen carbocyclischen Kern bilden, der gegebenenfalls durch eine oder mehrere der funktionellen aus Alkyl, Carboxylat, Perkarbonsäure, Sulfonat, Nitro, Chlor oder Brom ausgewählten Gruppen substituiert sein kann;
oder in der $R_1$ und $R_2$ je entweder Wasserstoff oder eine Alkylgruppe bedeuten und beide $R_0$ zusammen eine olefinische Bindung bilden;
und n die in der Verbindung vorhandene Anzahl von Carboxylatgruppen darstellt.

2. Zusammensetzung nach Anspruch 1, dadurch gekennzeichnet, daß das Brauseeffekt erzeugende System ein festes wasserlösliches Karbonat oder Bikarbonat zusammen mit einer festen wasserlöslichen organischen Säure oder anorganischen festen Säure oder einem Salz einer anorganischen Säure umfaßt.

3. Zusammensetzung nach Anspruch 1 oder 2, dadurch gekennzeichnet, daß die Säure aus Bernsteinsäure, Sulfamidsäure und einem Alkalibisulfat ausgewählt ist.

4. Zusammensetzung nach Anspruch 1, dadurch gekennzeichnet, daß der Brauseeffekt-Erzeuger ein wasserfreies Natriumperborat umfaßt.

5. Zusammensetzung nach einem der vorhergehenden Ansprüche, dadurch gekennzeichnet, daß der Brauseeffekt-Erzeuger 10 bis 40 Gew.-% der Zusammensetzung ausmacht.

6. Zusammensetzung nach einem der vorhergehenden Ansprüche, dadurch gekennzeichnet, daß sie bis 40 Gew.-% eines wasserlöslichen Alkalis, ausgewählt aus wasserlöslichen Alkalisikaten, -phosphaten, -boraten und -harbonaten enthält.

7. Zusammensetzung nach einem der vorhergehenden Ansprüche, dadurch gekennzeichnet, daß das hydratisierte Magnesiumsalz der Peroxyverbindung 5 bis 35 Gew.-% der Zusammensetzung ausmacht.

8. Zusammensetzung nach einem der vorhergehenden Ansprüche, dadurch gekennzeichnet, daß das hydratisierte Magnesiumsalz Magnesiummonoperoxyphthalat ist.

9. Zusammensetzung nach einem der vorhergehenden Ansprüche, dadurch gekennzeichnet, daß sie ausreichend Alkali enthält, sodaß die entstandene wässerige Lösung einen pH von über 9 hat und 1 bis 40 Gew.-% eines löslichen Erdalkalimetall-Komplexbildners enthält.

10. Zusammensetzung nach Anspruch 9, dadurch gekennzeichnet, daß der Komplexbildner aus Natriumcitrat, Natriumtartrat und Amino-poly-(methylenphosphonsäure)-salzen ausgewählt ist.

**Revendications**

1. Composition de nettoyage pour prothèses dentaires comprenant:

(1) au moins 10% en poids d'un générateur ou système générateur d'effervescence en particules, soluble dans l'eau,

(2) suffisamment d'acide ou d'alcali net, sous forme de particules, pour obtenir dans l'eau un pH de solution désiré, spécialement un pH choisi dans la gamme de pH 2 à pH 12, lors de la dissolution de la composition,

(3) au moins 1% en poids de particules d'un sel de magnésium hydraté d'un composé peroxygéné substitué ayant (à l'état anhydre) la formule empirique:

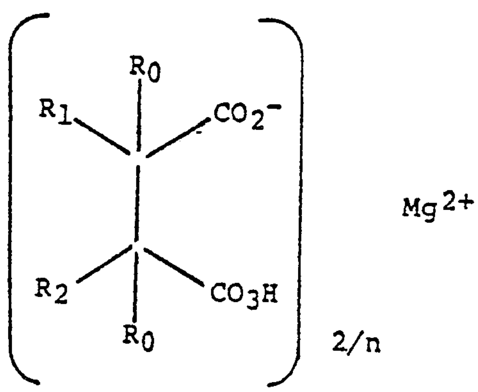

dans laquelle les $R_0$ représentent chacun de l'hydrogène ou se combinent l'un avec l'autre pour former une liaison oléfinique et $R_1$ et $R_2$ se combinent l'un avec l'autre et les atomes de carbone auxquels ils sont attachés pour former un noyau carbocyclique, ce noyau pouvant éventuellement être en outre encore substitué par un ou plusieurs des groupes fonctionnels choisis parmi les groupes alkyle, carboxylate, acide percarboxylique, sulfonate, nitro, chloro et bromo;
ou dans laquelle $R_1$ et $R_2$ représentent chacun de l'hydrogène ou un groupe alkyle et les deux $R_0$ se combinent l'un avec l'autre pour former une liaison oléfinique;
et n représente le nombre de groupes carboxylate présents dans le composé.

2. Composition suivant la revendication 1 caractérisé en ce que le système générateur d'effervescence comprend un carbonate ou bicarbonate solide soluble dans l'eau ainsi qu'un acide organique solide ou un acide inorganique solide ou un sel d'acide inorganique solide, tous solubles dans l'eau.

3. Composition suivant l'une des revendications 1 ou 2 caractérisée en ce que l'acide est choisi parmi l'acide succinique, l'acide sulfamique ou un bisulfate de métal alcalin.

4. Composition suivant la revendication 1 caractérisé en ce que le générateur d'effervescence comprend du perborate de sodium anhydre.

5. Composition suivant l'une quelconque des revendications précédentes caractérisé en ce que le générateur d'effervescence constitue 10 à 40% poids/poids de la composition.

6. Composition suivant l'une quelconque des revendications précédentes caractérisé contenant jusqu'à 40% poids/poids d'un alcali soluble dans l'eau choisi parmi les silicates, les phosphates, les borates et les carbonates de métal alcalin solubles dans l'eau.

7. Composition suivant l'une quelconque des revendications précédentes caractérisé en ce que le sel de magnésium hydraté du composé peroxygéné constitue 5 à 35% poids/poids de la composition.

8. Composition suivant l'une quelconque des revendications précédentes caractérisé en ce que le sel de magnésium hydraté est du monoperoxyphtalate de magnésium.

9. Composition suivant l'une quelconque des revendications précédentes caractérisé en ce qu'elle contient suffisamment d'alcali pour que la solution aqueuse résultante ait un pH supérieur à 9 et en ce qu'elle contient de 1 à 40% poids/poids d'un agent complexant soluble des métaux alcalino-terreux.

10. Composition suivant la revendication 9 caractérisée en ce que l'agent complexant est choisi parmi le citrate de sodium, le tartrate de sodium et les sels d'un acide amino-polyméthylène-phosphonique.